# Europäisches Patentamt
# European Patent Office
# Office européen des brevets

(11) Veröffentlichungsnummer: **0 169 398**
**B1**

(12) **EUROPÄISCHE PATENTSCHRIFT**

(45) Veröffentlichungstag der Patentschrift: 29.08.90

(51) Int. Cl.⁵: **A 61 K 9/48,** A 61 K 31/65

(21) Anmeldenummer: 85107900.4

(22) Anmeldetag: 26.06.85

(54) Oxytetracyclin-HC1-Weichgelatinekapseln und Verfahren zu ihrer Herstellung.

(30) Priorität: 24.07.84 DE 3427238

(43) Veröffentlichungstag der Anmeldung:
29.01.86 Patentblatt 86/05

(45) Bekanntmachung des Hinweises auf die
Patenterteilung:
29.08.90 Patentblatt 90/35

(84) Benannte Vertragsstaaten:
CH DE FR GB IT LI

(56) Entgegenhaltungen:
FR-A-2 126 443
US-A-2 867 661
US-A-2 915 555

ROTE LISTE, 1983, Bundesverband der
Pharmazeutischen Industrie e.V., Editio Cantor,
Aulendorf/Württ, DE

(73) Patentinhaber: R.P. Scherer GmbH
Gammelsbacher Strasse 2 Postfach 1243
D-6930 Eberbach/Baden (DE)

(72) Erfinder: Brox, Werner
Kätchen-Paulusstrasse 6
D-6124 Beerfelden (DE)
Erfinder: Burger, Artur, Prof.
Mariahilfpark 3
A-6020 Innsbruck (AT)

(74) Vertreter: Werner, Hans-Karsten, Dr. et al
Deichmannhaus am Hauptbahnhof
D-5000 Köln 1 (DE)

EP 0 169 398 B1

(56) Entgegenhaltungen:

**CHEMICAL ABSTRACTS, BAND 73, NR. 8, 24. AUGUST 1970, SEITE 221, ZUSAMMENFASSUNG NR. 38500H, COLUMBUS, OHIO, US; V. BALUTSOV: "HYGROSCOPIC AND NONHYGROSCOPIC FORMS OF OXYTETRACYCLINE HYDROCHLORIDE" CHEMICAL ABSTRACTS, BAND 98, NR. 4, 24. JANUAR 1983, SEITE 334, ZUSAMMENFASSUNG NR. 221894U, COLUMBUS, OHIO, US; L.K. GRAKOVSKAYA ET AL.: "EFFECT OF CRYSTALLINITY ON DISSOLUTION RATE OF OXYTETRACYCLINE HYDROCHLORIDE"**

**Beschreibung**

Gegenstand der vorliegenden Erfindung ist ein Verfahren zur Herstellung von Oxytetracyclin-HCl-Weichgelatinekapseln mit erhöhter Stabilität.

Oxytetracyclin-HCl wird seit vielen Jahren in erheblichem Umfang zu Gelatine-Kapseln verarbeitet. Neben Hartgelatine-Kapseln haben Weichgelatine-Kapseln eine erhebliche Bedeutung gewonnen, da man in sie außer dem Wirkstoff Oxytetracyclin-HCl ohne Schwierigkeiten auch andere feste und flüssige Wirkstoffe in die Kapsel miteinbringen kann.

Bei der Herstellung von Oxytetracyclin-HCl-Weichgelatinekapseln wurde von der Anmelderin festgestellt, daß die Kapseln in Abhängigkeit von den Rohstoffchargen zu einer Versprödung der Kapselhülle innerhalb weniger Wochen führen können, so daß bereits bei leichtem Fingernageldruck oder beim Transport als Bulkware feine Risse entstehen und die Kapseln undicht werden können.

Die Erfindung hat sich die Aufgabe gestellt, die Ursachen für das unterschiedliche Verhalten verschiedener Wirkstoffchargen zu untersuchen.

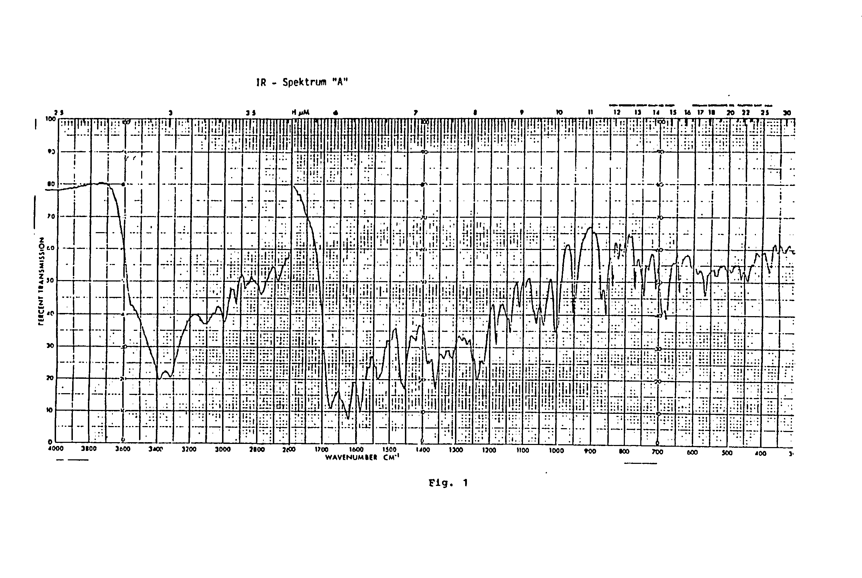

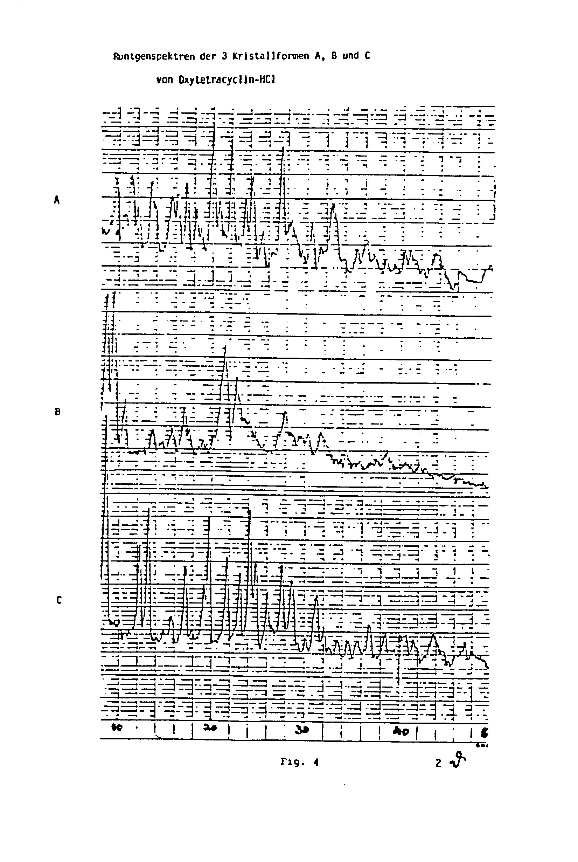

Im Rahmen dieser Untersuchungen wurde festgestellt, daß in der chemischen Reinheit der verschiedenen Wirkstoffchargen keine Unterschiede bestehen (alle Chargen entsprachen dem geltenden Arzneibuch bezüglich Reinheit), daß jedoch Oxytetracyclin-HCl in mindestens 3 Kristallformen kristallisieren kann, die sich in ihren physikalischen Eigenschaften wesentlich unterscheiden. Von diesen Kristallformen ist die Kristallform A welche das IR Spektrum "A" gemäß Fig. 1/4 und das Röntgenspektrum gemäß Fig. 4/4 A aufweist, in Weichgelatinekapseln die stabilste. Die Kristallform B kann sich in Weichgelatinekapseln innerhalb eines längeren Zeitraums in die Kristallform A umwandeln. Ähnlich wie Kristallform B verhält sich Kristallform C. Weitere Untersuchungen haben ergeben, daß die Handelsware in etwa der Hälfte der Fälle aus der Kristallform A und etwa zur Hälfte aus der Kristallform B besteht. Wirkstoffchargen, bestehend aus Kristallform C wurden nicht festgestellt.

Die 3 Kristallformen A, B und C unterscheiden sich insbesondere in ihren IR-Spektren, ihren Röntgendiffraktogrammen, ihrer Hygroskopizität und durch alle anderen physikalischen Eigenschaften, die die Folge unterschiedlicher Kristallgitter sind. Während die Kristallform A bei Raumtemperatur und nahe 100% relativer Luftfeuchtigkeit innerhalb von 2 Stunden weniger als 1% Wasser aufnimmt, nimmt die Kristallform B unter gleichen Bedingungen mehr als 8% Wasser auf. Es wurde weiterhin festgestellt, daß die Kristallform B in der Lage ist, während der Herstellung und Lagerung von Weichgelatinekapseln die Restfeuchtigkeit der Kapselhülle aufzunehmen und dadurch zu bewirken, daß die Weichgelatinekapseln verspröden. Die Kristallform A hingegen ist nicht in der Lage, der Weichgelatinekapsel Feuchtigkeit zu entziehen, so daß Oxytetracyclin-HCl-Weichgelatinekapseln, in denen die Kristallform A als Wirkstoff enthalten ist, hohe Stabilität aufweisen. Schließlich wurde gefunden, daß die Kristallform B nach Aufnahme einer ausreichenden Menge von Wasser sich im Laufe der Zeit nicht voraussehbar in die Kristallform A umwandeln kann und dabei die aufgenommene Feuchtigkeit wieder an die Kapselhülle abgibt. Dies erklärt die von der Anmelderin gemachte Beobachtung, daß spröde Kapseln nach längerer Lagerung gelegentlich ihre Sprödigkeit wieder verlieren.

In der Literatur werden 2 Kristallformen beschrieben, die sich in ihren hygroskopischen Eigenschaften unterscheiden sollen [Balutsov., V., Farmatsiya (Sofia) 20 (1) 34 (1970); Grakovskaya, L. K., Nesterova, L. Ya., Antibiot. (Moskau) 27 (11) 815 (1982)]. In diesen Arbeiten sind auch Daten von Röntgenspektren bzw. Fotografien einer hygroskopischen und nicht hygroskopischen Form angegeben.

Die U.S. Patentschrift 2867661 beschreibt eine Methode zur Herstellung einer wenig hygroskopischen kristallinen Modifikation des Oxytetracyclin Hydrochlorides. Nähere Hinweise auf Herstellungsverfahren, Umwandlungsverhalten und Auswirkungen auf die pharmazeutische Technologie insbesondere auf die Stabilität von Weichgelatinekapseln lassen sich aus dem Stand der Technik nicht entnehmen.

Auch sind aus der Roten Liste, 1983, Nr. 10180, 10181 Oxytetracyclin-HCl Kapseln bekannt. Die weitere Untersuchung und Charakterisierung der Kristallformen von Oxytetracyclin-HCl hat zu der Erkenntnis geführt, daß diese in erheblichem Maße Einfluß nehmen auf die Stabilität von Weichgelatinekapseln. Versuche, das Verspröden der Kapselhülle von Kristallform B enthaltenden Weichgelatinekapseln durch die üblichen technologischen Maßnahmen, wie z.B. Erhöhung des Gehaltes an Glycerin oder Sorbit (Weichmacher) in der Kapselhülle zu verhindern, schlugen fehl. Es wurde nämlich überraschend gefunden, daß die Kristallform B in Weichgelatinekapseln nicht stabil ist und sich im Laufe der Zeit — nicht voraussehbar — in die Kristallform A umwandelt. Dadurch erweicht die Kapselhülle völlig, die Kapseln werden deformiert und unansehnlich und sind damit nicht handelsfähig. Dagegen sind Weichgelatinekapseln mit Kristallform A auch nach jahrelanger Lagerung stabil und handelsfähig.

Gegenstand der vorliegenden Erfindung ist somit ein Verfahren zur Herstellung von Oxytetracyclin-HCl-Weichgelatinekapseln mit erhöhter Stabilität, dadurch gekennzeichnet, daß man von dem Oxytetracyclin-HCl zunächst die geeignete nicht hygroskopische Kristallform A welche das IR Spektrum "A" gemäß Fig. 1/4 und das Röntgenspektrum gemäß Fig. 4/4 A aufweist, herstellt bzw. identifiziert und/oder die Wasseraufnahme aus feuchter Luft bestimmt und nur solche Chargen zur Herstellung von Weichgelatinekapseln auswählt, die aus der nicht hygroskopischen Kristallform A bestehen.

Die Herstellung von Weichgelatine-Kapseln erfolgt erfindungsgemäß in an sich bekannter Weise, vorzugsweise nach dem von der Anmelderin selbst entwickelten und publizierten Verfahren zur Herstellung von Weichgelatine-Kapseln. Als Hilfsstoffe und weitere Wirkstoffe können erfindungsgemäß alle bisher für Oxytetracyclin-HCl verwendeten Hilfsstoffe und Wirkstoffe verwendet werden. In allen Fällen ist es ausschließlich entscheidend, daß das eingesetzte Oxytetracyclin-HCl aus der Kristallform A welche das IR Spektrum "A" gemäß Fig. 1/4 und das Röntgenspektrum gemäß Fig. 4/4 A aufweist, besteht. Falls die für die Herstellung stabiler Weichgelatinekapseln notwendige Kristallform A nicht zur Verfügung steht, kann sie aus Kristallform B oder Kristallform C auf folgende Weise gewonnen werden. Zur Darstellung der Form A kommen verschiedene Möglichkeiten in Betracht. Im folgenden werden der Einfachheit halber nur solche beschrieben, bei denen ausschließlich Oxytetracyclin-HCl, also nicht auch die freie Base, als Ausgangsmaterial in Betracht kommt, und bei denen das Herstellungsverfahren unabhängig von der eingesetzten Kristallform ist.

Oxytetracyclinhydrochlorid wird mit etwa der 7- bis 10-fachen Menge eines Lösungsmittels vermengt, in dem Oxytetracyclin-HCl relativ schlecht aber nicht unlöslich ist. Diese Suspension wird ungefähr 1 bis 1,5 Stunden im Rotationsverdampfer unter Rückfluß gekocht bzw. auf etwa 60°C erwärmt oder gleich lange in einem hermetisch schließendem Gefäß bei etwa 60°C gerührt (Magnetrührer). Die noch heiße Suspension wird filtriert und das Kristallisat unter einem Druck von unter 1 mbar bei 60°C getrocknet. Als geeignete Lösungsmittel für diese Art des Herstellungsverfahrens haben sich beispielsweise Aceton, Propanol-1 und Propanol-2 erwiesen.

Hingegen erhält man Kristallform B z.B. aus 0,1 bis 0,2N HCl in Methanol und Kristallform C aus 96 %igem Ethanol oder Methanol durch übliche Umkristallisation.

Im Gegensatz zur Form A, die sich durch ein eher dunkles Gelb auszeichnet, ist B leuchtend schwefelgelb und C fast weißgelb.

Aus den nachfolgenden Versuchen, Vergleichsversuchen und experimentellen Daten gehen weitere Einzelheiten der Kristallform A, B und C hervor sowie ihre Bedeutung für die Stabilität von Weichgelatinekapseln.

Versuch 1 (IR-Spektren)

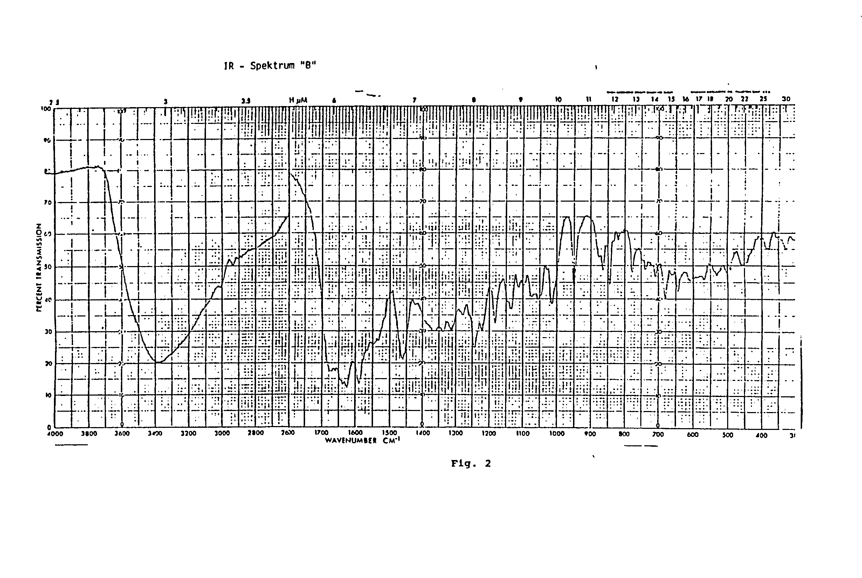

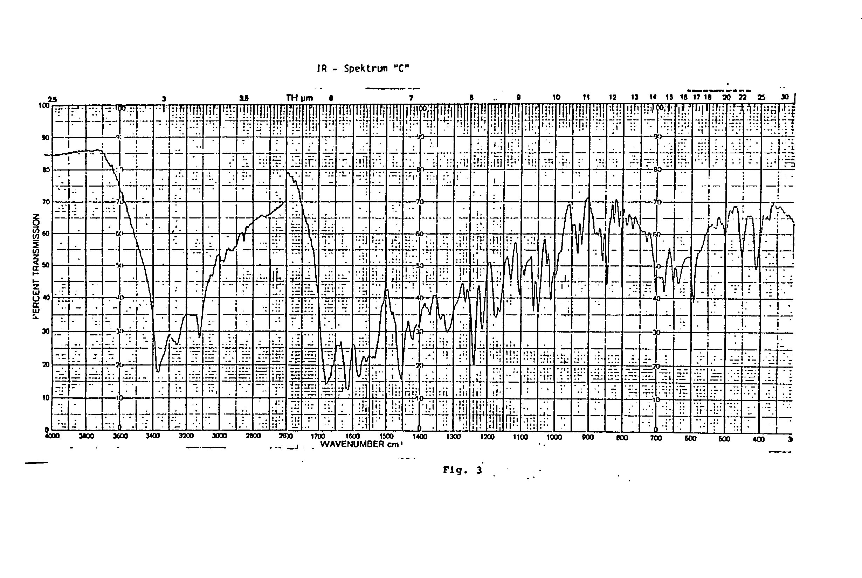

Proben von Kristallform A, B und C wurden getrocknet (60°C, 1 mbar, 48 Stunden) und von Kristallform A, B und C anschließend IR-Spektren angefertigt. Diese Spektren sind in den Fig. 1 bis 3 wiedergegeben. Das IR-Spektrum der Kristallform A ist ähnlich dem aus der Literatur bekannten Spektrum; vgl. "UV- und IR-Spektrum wichtiger pharmazeutischer Wirkstoffe", von H.-W. Dibbern, Editio Cantor, Aulendorff (1983).

Versuch 2 (Röntgenspektren)

Von allen 3 Kristallformen wurden Röntgenbeugungsaufnahmen angefertigt. (Cu, K$_\alpha$-Strahlung, Zählrohrbewegung 1 gd/min). Die Spektren sind in Fig. 4 wiedergegeben.

Versuch 3 (Herstellung von Oxytetracyclin-HCl-Weichgelatinekapseln mit Oxytetracyclin-HCl der Kristallform A, B und C)

Es wurden handelsübliche Oxytetracyclin-HCl-Weichgelatinekapseln nach dem Scherer-Rotary-Die-Verfahren mit Oxytetracyclin-HCl der Kristallformen A, B und C unter Zusatz von Miglyol 812[R], Bienenwachs, hydrierten Pflanzenölen und Sojalecithin hergestellt. Die Kapselhülle bestand aus 45% Gelatine, 22% Glycerin 85 %ig und 33% Wasser. Pro Kapsel wurden 540 mg Oxytetracyclin-HCl dosiert. Das Kapselformat war 14 minims oblong (1 minim = 0,0616 ml). Die Trocknung der Kapseln erfolgte bei 20°C und einer relativen Luftfeuchtigkeit von 20%.

Es wurden jeweils nach 1, 2, 5, 7 und 9 Tagen Trocknungszeit Kapselmuster entnommen. Die IR-spektroskopische Prüfung ergab zu diesem Zeitpunkt noch keine Kristallformänderung. Die Kapseln wurden 6 Monate in Glasflaschen gelagert und anschließend das IR-Spektrum von Oxytetracyclin-HCl erneut aufgenommen. Außerdem wurde der Wassergehalt der Kapselhülle und der Wassergehalt des Oxytetracyclin-HCl in der Kapselfüllung bestimmt. Um den Einfluß der Temperatur auf das Umwandlungsverhalten zu untersuchen, wurden die 9 Tage getrockneten Kapseln zusätzlich bei 37°C und 45°C gelagert und nach 6 Wochen sowie nach 3 Monaten die Kristallform IR-spektroskopisch überprüft. Ferner wurde der Wassergehalt der Kapselhülle und der Wassergehalt des Oxytetracyclin-HCl im Inhalt analysiert.

Alle Kapselmuster wurden durch Druckprüfung an der Kapselnaht auf Sprödigkeit untersucht. Bei spröden Kapseln ist ein deutliches Knacken der Gelatinehülle wahrnehmbar. Diese empirische Methode hat sich in der Praxis oft als aussagekräftiger erwiesen als Härte- und Biegefestigkeitsmessungen. Von den 9 Tage getrockneten Kapseln wurden zusätzlich Härtetests durchgeführt.

Das Umwandlungsverhalten der Kristallformen in Abhängigkeit der Lagerungsbedingungen und der Trocknungszeit bzw. des Feuchtigkeitsgehaltes der Kapseln ist in der Tabelle 1 aufgeführt.

Die Kristallform A ist unter allen Bedingungen völlig stabil. Der Wassergehalt an Oxytetracyclin-HCl ist wegen der fehlenden Hygroskopizität von A auch bei kurzer Trocknungszeit sehr niedrig. Zu keinem Zeitpunkt konnte eine Versprödung der Kapselhülle festgestellt werden.

Die Kristallform B ist dagegen in Weichgelatinekapseln nicht stabil. Die IR-spektroskopische Prüfung ergab, daß sich bei den nur 1 Tag getrockneten Kapseln nach 6 Monaten bei Raumtemperatur die Form B in A umgewandelt hat. Bei den länger getrockneten Kapseln lag nach dieser Zeit noch die Form B vor. Bei den 9 Tage getrockneten und 3 Monate bei 37°C sowie 6 Wochen oder 3 Monate bei 45°C gelagerten Kapseln hat sich die Form B bereits in A umgewandelt, was darauf hinweist, daß sich früher oder später alle Muster der Form B in A umwandeln werden.

EP 0 169 398 B1

Die Wassergehaltsbestimmungen der Kapselhülle und des Oxytetracyclin-HCl in der Füllung zeigen, daß der Wassergehalt des Oxytetracyclin-HCl bei der hygroskopischen Form B mit ca. 5% im Vergleich zu den Formen A und C recht hoch ist, während der Wassergehalt in der Kapselhülle am niedrigsten ist. Sobald sich jedoch die hygroskopische Form B in die nicht hygroskopische Form A umlagert, nimmt der Wassergehalt des Oxytetracyclin-HCl deutlich ab. Der Wassergehalt der Kapselhülle steigt gleichzeitig an, da das Wasser aus der Füllung infolge der Hygroskopizität des Weichmachers Glycerin in die Hülle aufgenommen wird.

Die Sprödigkeitsuntersuchungen ergaben, daß die Kapseln direkt nach der Herstellung zunächst nur etwas spröde waren. Mit zunehmender Lagerzeit verstärkte sich die Versprödung der Kapselhülle. Nach 6 Monaten Lagerung waren alle Kapselmuster, in denen die Form B noch unverändert vorlag, sehr spröde, während bei den Mustern, bei denen sich B in A umgewandelt hatte, keine Sprödigkeit mehr festzustellen war. In Übereinstimmung mit den Sprödigkeitsuntersuchungen zeigten die Härtemessungen einen deutlichen Anstieg der Kapselhärte während der 6-monatigen Lagerung.

Bei den Kapseln mit der Kristallform C wurde bei der IR-spektroskopischen Prüfung keine Umwandlung der Kristallform festgestellt. Der Wassergehalt des Oxytetracyclin-HCl war bei den unterschiedlich lange getrockneten Kapseln praktisch gleich und ähnlich wie bei A Sehr niedrig. Die Einbettung in eine ölige Hilfsstoffmischung verhindert offenbar die Wasseraufnahme aus der noch feuchten Gelatinehülle der gegenüber A hygroskopischeren und gegenüber B weniger hygroskopischen Form C.

Die Untersuchungen auf Sprödigkeit zeigten überraschenderweise, daß die Kapseln direkt nach der Herstellung und Trocknung deutlich spröde waren, daß die Sprödigkeit der Kapseln während der Lagerung in Übereinstimmung mit den Härtewerten jedoch wieder abnahm. Nach 6 Monaten waren die Kapseln nicht mehr spröde. Die Ursachen für dieses Verhalten sind nicht bekannt. Das Erweichen der Kapseln ist möglicherweise auf die Abgabe von Ethanol aus der Form C an die Kapselhülle zurückzuführen.

| Trocknungszeit | Lagerung | Veränderung der Kristallform | $H_2O$-Gehalt der Kapselhülle % | $H_2O$-Gehalt im Füllgut % |
|---|---|---|---|---|
| Kristallform A | | | | |
| 1 Tag | 6 Monate RT | — | 9,9 | 0,9 |
| 2 Tage | 6 Monate RT | — | 9,1 | 0,7 |
| 5 Tage | 6 Monate RT | — | 8,4 | 0,6 |
| 7 Tage | 6 Monate RT | — | 7,5 | 0,5 |
| 9 Tage | 6 Monate RT | — | 6,9 | 0,5 |
| 9 Tage | 6 Wochen 37°C | — | 6,5 | 0,6 |
| 9 Tage | 3 Monate 37°C | — | 6,2 | 0,6 |
| 9 Tage | 6 Wochen 45°C | — | 5,8 | 0,5 |
| 9 Tage | 3 Monate 45°C | — | 5,8 | 0,6 |

5

Kristallform B

| | | | | |
|---|---|---|---|---|
| 1 Tag | 6 Monate RT | A | 12,9 | 1,0 |
| 2 Tage | 6 Monate RT | — | 7,3 | 5,3 |
| 5 Tage | 6 Monate RT | — | 6,8 | 5,3 |
| 7 Tage | 6 Monate RT | — | 6,2 | 5,2 |
| 9 Tage | 6 Monate RT | — | 5,6 | 4,9 |
| 9 Tage | 6 Wochen 37°C | — | 6,0 | 4,9 |
| 9 Tage | 3 Monate 37°C | A | 9,7 | 1,1 |
| 9 Tage | 6 Wochen 45°C | A | 8,6 | 0,9 |
| 9 Tage | 3 Monate 45°C | A | 8,8 | 1,1 |

Kristallform C

| | | | | |
|---|---|---|---|---|
| 1 Tag | 6 Monate RT | — | 11,4 | 0,7 |
| 2 Tage | 6 Monate RT | — | 9,1 | 0,6 |
| 5 Tage | 6 Monate RT | — | 9,0 | 0,6 |
| 7 Tage | 6 Monate RT | — | 7,5 | 0,7 |
| 9 Tage | 6 Monate RT | — | 7,2 | 0,7 |
| 9 Tage | 6 Wochen 37°C | — | 7,5 | 0,8 |
| 9 Tage | 3 Monate 37°C | — | 6,8 | 0,7 |
| 9 Tage | 6 Wochen 45°C | — | 7,0 | 0,8 |
| 9 Tage | 3 Monate 45°C | — | 6,7 | 0,9 |

Versuch 4 (Wasseraufnahme der Kristallform A, B und C)

Muster der Kristallform A und B wurden bezüglich ihres Wassergehaltes (Karl-Fischer-Titration) vor und nach der Lagerung nach 2 Stunden nahe 100% relativer Luftfeuchtigkeit untersucht. Die Ergebnisse sind in der nachfolgenden Tabelle zusammengestellt.

| | $H_2O$-Gehalt in % (Ausgangswert) | nach 2 Std. nahe 100% Feuchte/RT | Wasseraufnahme in % |
|---|---|---|---|
| Kristallform A | 0,7 | 1,4 | 0,7 |
| Kristallform B | 1,2 | 9,7 | 8,5 |
| Kristallform C | 1,9 | 8,5 | 6,6 |

**Patentanspruch**

Verfahren zur Herstellung von Oxytetracyclin-HCl-Weichgelatinekapseln mit erhöhter Stabilität, dadurch gekennzeichnet, daß man von dem Oxytetracyclin-HCl zunächst die geeignete nicht hygroskopische Kristallform A welche das IR Spektrum "A" gemäß Fig. 1/4 und das Röntgenspektrum gemäß Fig. 4/4 A aufweist, herstellt bzw. identifiziert und/oder die Wasseraufnahme aus feuchter Luft bestimmt und nur solche Chargen für die Herstellung von Kapseln auswählt, die aus der nicht hygroskopischen Kristallform A bestehen.

6

## EP 0 169 398 B1

**Revendication**

Procédé d'obtention de capsules de gélatine molle d'oxytétracycline — HCl, à stabilité améliorée, caractérisé en ce qu'on obtient d'abord la forme cristalline A non hygroscopique convenable de l'oxytétracycline-HCL, qui présente le diagramme de spectre infrarouge A de la figure 1/4 et le diagramme de rayons X de la figure 4 A, ou on l'identifie, et/ou on détermine l'absorption d'eau à partir d'air humide, et on choisit pour la fabrication de capsules des charges qui sont formées de la forme cristalline A, qui n'est pas hygroscopique.

**Claim**

A process for the preparation of oxytetracycline-HCl soft gelatin capsules having an increased stability, characterized in that first the non-hygroscopic crystal form A of oxytetracyline-HCl is prepared and/or identified, said crystal form having the IR spectrum "A" according to Figure 1/4 and the X-ray spectrum according to Figure 4/4, and/or the water absorption from moist air is determined and, only those batches consisting of the non-hygroscopic crystal form A are selected for the preparation of the capsules.

IR - Spektrum "A"

Fig. 1

IR - Spektrum "B"

Fig. 2

IR – Spektrum "C"

Fig. 3

# EP 0 169 398 B1

Röntgenspektren der 3 Kristallformen A, B und C

von Oxytetracyclin-HCl

A

B

C

Fig. 4

2 ϑ

4